(19) Europäisches Patentamt

European Patent Office

Office européen des brevets

(11) EP 0 993 865 A2

(12) EUROPÄISCHE PATENTANMELDUNG

(43) Veröffentlichungstag:
19.04.2000 Patentblatt 2000/16

(51) Int. Cl.⁷: **B01J 23/04**, B01J 21/06,
C07C 2/72, C07C 209/60,
C07C 2/24, C07C 5/25

(21) Anmeldenummer: **99119144.6**

(22) Anmeldetag: **05.10.1999**

(84) Benannte Vertragsstaaten:
**AT BE CH CY DE DK ES FI FR GB GR IE IT LI LU MC NL PT SE**
Benannte Erstreckungsstaaten:
**AL LT LV MK RO SI**

(30) Priorität: **09.10.1998 DE 19846549**

(71) Anmelder:
**BASF AKTIENGESELLSCHAFT**
**67056 Ludwigshafen (DE)**

(72) Erfinder:
• **Steinbrenner, Ulrich, Dr.**
**67063 Ludwigshafen (DE)**
• **Narbeshuber, Thomas, Dr.**
**49477 Ibbenbürenen (DE)**

(74) Vertreter:
**Isenbruck, Günter, Dr. et al**
**Patent- und Rechtsanwälte,**
**Bardehle-Pagenberg-Dost-Altenburg-Geissler-Isenbruck**
**Theodor-Heuss-Anlage 12**
**68165 Mannheim (DE)**

(54) **Basischer Katalysator auf Basis von Titanaten, Zirkonaten und Hafnaten**

(57) Der Katalysator enthält mindestens ein Alkalimetall auf einem Träger, der durch die allgemeine Formel (I) charakterisiert ist,

$$A_{n'}Ti_{m'}Zr_{p'}Hf_{q'}O_{n'+2(m'+p'+q')} \qquad (I)$$

mit der Bedeutung von
A zweiwertiges Metall,

$$20 \cdot (m'+p'+q') > n' > 0,05 \cdot (m'+p'+q'),$$

der mit mindestens einer Verbindung eines Alkalimetalls und/oder Erdalkalimetalls dotiert sein kann,
wobei das Gewichtsverhältnis Alkalimetall/Träger 0,01 bis 5 zu 1 und bei Vorliegen einer Dotierung das Gewichtsverhältnis Dotierung/Träger 0,01 bis 5 zu 1 beträgt und wobei der Phasenanteil des Trägers, der einer $ZrO_2$- oder einer Erdalkalioxid-Struktur entspricht oder aus $ZrO_2$ oder Erdalkalioxid besteht, kleiner als 10 Gew.-% beträgt.

Der Katalysator wird zur Seitenkettenalkylierung oder -alkenylierung von alkylaromatischen Verbindungen mit Olefinen oder Diolefinen, zur Doppelbindungsisomerisierung von Olefinen, zur Dimerisierung von Olefinen, zur Kern-Kern-Kopplung von Aromaten sowie zur Aminierung von Olefinen und konjugierten Diolefinen verwendet.

EP 0 993 865 A2

**Beschreibung**

Die Erfindung betrifft einen Katalysator, dessen Verwendung in stark basisch katalysierten Reaktionen und ein Verfahren zur Seitenkettenalkylierung oder -alkenylierung von alkylaromatischen oder alkylalicyclischen Verbindungen mit Olefinen oder Diolefinen.

Die Seitenkettenalkylierung, insbesondere von aromatischen Verbindungen, die ein acides Proton in der $\alpha$-Position der Seitenkette tragen, in Gegenwart von Katalysatoren ist bekannt.

In der EP-B-0 439 679 ist ein Verfahren zur Alkylierung von alkylaromatischen Kohlenwasserstoffen beschrieben. Die Umsetzung erfolgt in Gegenwart eines Katalysators aus aktiviertem Aluminiumoxid, das mit Magnesiumhydroxid und Kaliummetall dotiert ist. Anstelle von Magnesiumhydroxid kommen auch Calciumhydroxid, Bariumhydroxid oder Magnesiumoxid zum Einsatz. Auch eine Imprägnierung mit Kaliumhydrid ist beschrieben.

US 4,914,250 betrifft ein Verfahren zur Seitenkettenalkylierung von Aromaten. Als Katalysator wurde dabei Diatomeenerde eingesetzt, die im Reaktionsgemisch neben Kalium oder NaK und Spuren von Wasser vorlag.

US 4,922,054 betrifft ebenfalls ein Verfahren zur Seitenkettenalkylierung von Aromaten, bei dem ebenfalls als Katalysator Diatomeenerde eingesetzt wurde, die im Reaktionsgemisch neben NaK und Kaliumoxid vorlag. Anstelle von Kaliumoxid wurde auch Rubidiumoxid verwendet. Anstelle von NaK wurde auch Kaliummetall eingesetzt.

JP-A-05 163 171 betrifft die Herstellung von Alkenylbenzol und seinen Derivaten. Der verwendete Katalysator umfaßt ein Alkalimetall und ein Kaliumcarbonatsalz und/oder KOH, die in Gegenwart eines Olefins und/oder Diolefins dispergiert werden. Vorzugsweise wird als Alkalimetall, Natriummetall und als Kaliumcarbonatsalz $K_2CO_3$, $KHCO_3$ oder $KNaCO_3$ eingesetzt.

Gemäß EP-B-0 575 724 wird ein Katalysator eingesetzt, der durch Tränken von Zirkoniumoxidpulver oder Kaliumzirkonat mit Kaliumhydroxidlösung, anschließendem Calcinieren in Luft bei 500°C und Aufbringen von metallischem Natrium auf den Träger hergestellt wird. Der Katalysator wird zur Seitenkettenalkenylierung von o-Xylol mit Butadien eingesetzt.

Gemäß EP-A-0 636 597 wird ein Verfahren zur Seitenkettenalkenylierung von o-Xylol mit Butadien beschrieben, wobei als Katalysator Aluminiumoxid, Calciumoxid oder Zirkoniumoxid verwendet wird, das jeweils mit wäßriger KOH imprägniert ist und danach bei einer Temperatur von 500 bis 550°C calciniert wurde. Der Träger wird mit metallischem Natrium beschichtet.

Diese bisher bekannten Katalysatoren sind in ihrer Leistung für viele Anwendungen unzureichend. Zum einen sind die bislang bekannten Katalysatoren wenig aktiv, das heißt die Raum-Zeit-Ausbeute ist sehr klein. Zudem bilden sie bei hohen Umsätzen und längeren Standzeiten unerwünschte Folgeprodukte der zunächst erhaltenen Primärprodukte. Beispielsweise treten bei der Seitenkettenalkylierung von Toluol mit Propen nach der Bildung von Isobutylbenzol Cyclisierungen zu Methylindan auf, wie auch Dimerisierung des Olefins, beispielsweise Bildung von Methylpenten aus Propen. Weiterhin ist die Standzeit der beschriebenen Katalysatoren begrenzt. Die Katalysatoren verlieren mit zunehmender Reaktionszeit ihre Aktivität und ändern teilweise ihr Nebenproduktespektrum.

Aufgabe der vorliegenden Erfindung ist die Bereitstellung eines Katalysators für die Seitenkettenalkylierung oder -alkenylierung, der die Nachteile der bekannten Katalysatoren vermeidet und eine hohe Aktivität, Selektivität und Standzeit aufweist.

Die Aufgabe wird erfindungsgemäß durch Bereitstellung eines Katalysators gelöst, enthaltend mindestens ein Alkalimetall auf einem Träger charakterisiert durch die allgemeine Formel (I)

$$A_{n'}Ti_{m'}Zr_{p'}Hf_{q'}O_{n'+2(m'+p'+q')} \tag{I}$$

mit der Bedeutung von
A zweiwertiges Metall,

$$20 \cdot (m'+p'+q') > n' > 0,05 \cdot (m'+p'+q'),$$

der mit mindestens einer Verbindung eines Alkalimetalls und/oder Erdalkalimetalls dotiert sein kann, wobei das Gewichtsverhältnis Alkalimetall/Träger 0,01 bis 5 zu 1 und bei Vorliegen einer Dotierung das Gewichtsverhältnis Dotierung/Träger 0,01 bis 5 zu 1 beträgt und wobei der Phasenanteil des Trägers, der einer $ZrO_2$- oder einer Erdalkalioxid-Struktur entspricht oder aus $ZrO_2$ oder Erdalkalioxid besteht, kleiner als 10 Gew.-% beträgt.

Ferner wird die Aufgabe gelöst durch Verwendung dieses Katalysators in stark basisch katalysierten Reaktionen, vorzugsweise zur Seitenkettenalkylierung oder Seitenkettenalkenylierung von alkylaromatischen oder alkylalicyclischen Verbindungen mit Olefinen oder Diolefinen, zur Doppelbindungsisomerisierung von Olefinen oder zur Dimerisierung von Olefinen.

Die Aufgabe wird ferner beispielhaft gelöst durch Bereitstellung eines Verfahrens zur Seitenkettenalkylierung oder Seitenkettenalkenylierung von alkylaromatischen Verbindungen durch Umsetzung mit Olefinen oder Diolefinen, wobei die Umsetzung in Gegenwart eines vorstehend definierten Katalysators durchgeführt wird.

Das Verhältnis Alkalimetall/Träger beträgt dabei vorzugsweise 0,01 bis 2, besonders bevorzugt 0,01 bis 1 zu 1. Das Alkalimetall ist dabei vorzugsweise Natrium oder Kalium, insbesondere Natrium. Auch Gemische mehrerer Alkalimetalle können eingesetzt werden.

Der Träger besteht im undotierten Zustand aus komplexen Oxiden von zweiwertigen Metallen A und vierwertigem Titan, Zirkonium und/oder Hafnium. Solche Oxide sind z.B. Titanate der allgemeinen Formel $ATi_mO_{1+2m}$ wie $ATiO_3$, $ATi_2O_5$, $ATi_3O_7$, $ATi_4O_9$, $ATi_5O_{11}$ oder $ATi_6O_{13}$, oder der allgemeinen Formel $A_{1+m}Ti_mO_{1+3m}$ (d.h. n = 1+m ), wie $A_2TiO_4$ oder $A_3Ti_2O_7$, aber auch Verbindungen, die keiner der beiden, durch die vorstehenden allgemeinen Formeln definierten homologen Reihen angehören, wie $A_6Ti_{17}O_{40}$. Weitere Oxide sind auch die den Titanaten entsprechenden Verbindungen des vierwertigen Zirkoniums bzw. Hafniums (Zirkonate bzw. Hafnate) sowie die entsprechenden Verbindungen von zwei, bevorzugt von Zirkon und Haffnium oder von Titan und Zirkon, oder allen drei der genannten vierwertigen Metalle (wobei die allgemeinen Formeln dann den vorigen entsprechen), wie $ATi_2ZrO_7$, $ATiZr_2O_7$, $AZr_2HfO_7$ oder $A_3ZrHfO_7$. Vorzugsweise ist A ausgewählt aus Mg, Ca, Sr, Ba, Mn, Fe, Co, Ni, Pb, Zn, Cd, Pb und Gemischen davon, besonders bevorzugt aus Mg, Ca, Sr, Ba und Gemischen davon.

Typische Vertreter der Titanate sind $ATiO_3$ mit A = Mg, Ca, Sr, Ba, Fe, Cd und Zn, wie $MgTiO_3$ und $CaTiO_3$, $ATi_2O_5$ mit A= Mg, Ca, Sr, Ba, Co und Fe, wie $MgTi_2O_5$, ferner $ATi_3O_7$, $ATi_4O_9$, $ATi_5O_{11}$, $ATi_6O_{13}$, $A_2TiO_4$ und $A_3Ti_2O_7$, mit A = Mg, Ca, Sr, und Ba, wie $CaTi_3O_7$, wie $MgTi_2O_5$, $BaTi_4O_9$, $Sr_3Ti_2O_7$ und $Ba_4Ti_{13}O_{30}$.

Typische Vertreter der Zirkonate sind $AZrO_3$ mit A = Mg, Ca, Sr und Ba und Pb wie $CaZrO_3$ und $BaZrO_3$, $AZr_4O_9$ und $A_3Zr_2O_7$ mit A = Mg, Ca, Sr und Ba wie $BaZr_4O_9$ und $Sr_3Zr_2O_7$.

Typische Vertreter der Hafnate sind $AHfO_3$ mit A=Mg, Ca, Sr, Ba und Pb, $AHf_4O_9$ und $A_3Hf_2O_7$ mit A=Mg, Ca, Sr und Ba.

Typische Vertreter von Verbindungen des Titans und des Zirkons bzw. des Zirkons und des Hafniums sind beispielsweise $CaTiZr_2O_7$ und $CaTi_2ZrO_7$ bzw. $CaZrHf_2O_7$ und $CaZr_2HfO_7$.

Unter dem Begriff "Träger charakterisiert durch die allgemeine Formel (I)" ist zu verstehen, daß der Träger entweder aus einer der vorstehend dargestellten Verbindungen allein oder aus einem definierten Gemisch daraus besteht, d.h. in den einzelnen Verbindungstypen bzw. in der einzelnen Verbindung die Indices n, m, p, q und bei Vorliegen von Zr und Hf p+q (wobei m+p+q niemals 0 sein darf) zwar ganze Zahlen sind, aber über den gesamten Träger gesehen, in der ihn als ganzes charakterisierenden Formel (I), die Indices n', m', p', q' gemittelte Werte über alle Einzelwerte einzelner Verbindungstypen darstellen, d.h. - von Ausnahmen abgesehen - keine ganzen Zahlen sind. Im Grenzfall des Vorliegens einer einzelnen Verbindung sind n, m, p, q gleich mit n', m', p', q' und damit ganze Zahlen.

Vorzugsweise sind in dem Katalysator die Oxide der allgemeinen Formeln $ATi_mO_{1+2m}$, $A_nTi_mO_{n+2m}$, $AZr_pO_{1+2p}$, $A_nZr_pO_{n+2p}$, $ATi_mZr_pO_{1+2(m+p)}$, $A_nTi_mZr_pO_{n+2(m+p)}$ enthalten, mit der Bedeutung von n, m, p, q als ganze Zahlen.

Der Träger kann ferner mit mindestens einer Verbindung eines Alkalimetalls und/oder Erdalkalimetalls im Gewichtsverhältnis Dotierung/Träger von 0,01 bis 5, vorzugsweise 0,01 bis 2, insbesondere 0,01 bis 1 zu 1 dotiert sein. Vorzugsweise ist der Katalysator so dotiert. Die Dotierung der Träger erfolgt dabei insbesondere mit löslichen Verbindungen der Alkalimetalle und/oder Erdalkalimetalle, wie den Oxiden, Hydroxiden, Carbonaten, Formiaten, Acetaten und/oder Oxalaten. Bevorzugt werden die Hydroxide oder Carbonate, besonders bevorzugt $K_2CO_3$ und/oder KOH eingesetzt.

Der Phasenanteil des Trägers mit einer kristallographischen Phase, die einer $ZrO_2$- oder einer Erdalkalioxid-Struktur entspricht oder aus $ZrO_2$ oder Erdalkalioxid besteht, in den erfindungsgemäßen Trägern ist < 10 Gew.-%, wobei unter den genannten Strukturen zu verstehen ist, daß in der allgemeinen Formel (I) dann die Größen der Indices n', m', q' im Verhältnis zu p' bei einer $ZrO_2$- bzw. m', p', q' zu n' bei einer Erdalkalioxid-Struktur zu vernachlässigen sind.

## Herstellung der Katalysatoren

Die Katalysatoren werden hergestellt durch

- Aufbringen mindestens eines Alkalimetalls auf den Träger der allgemeinen Formel (I) durch
  Trägerung des schmelzflüssigen Alkalimetalls, oder
  Imprägnierung oder Tränkung des Trägers mit Lösungen eines Alkalimetallazids, Trocknen des so behandelten Trägers und Zersetzung des Alkalimetallazids, oder
  Aufdampfen des Alkalimetalls auf den Träger, oder
  Imprägnierung oder Tränkung des Trägers mit ammoniakalischen Lösungen des Alkalimetalls und Entfernen des Animoniaks vom so behandelten Träger,

- wobei der Träger der allgemeinen Formel (I) gegebenenfalls vorher durch Imprägnieren oder Tränken mit einer Lösung mindestens einer Verbindung eines Alkalimetalls und/oder Erdalkalimetalls, Trocknen und Calcinieren des dotierten Trägers dotiert wurde.

Die gegebenenfalls auszuführende Dotierung erfolgt dabei in an sich bekannter Weise durch Imprägnierung oder Tränkung und anschließende Calcinierung bei Temperaturen im Bereich von 100 bis 1500°C, vorzugsweise 250 bis 1000°C, besonders bevorzugt 250 bis 350°C. Dabei kann die Imprägnierung oder Tränkung mit einer Lösung der Verbindung des Alkalimetalls und/oder Erdalkalimetalls in einem beliebigen geeigneten Lösungsmittel erfolgen. Vorzugsweise werden wäßrige Lösungen eingesetzt, wobei nach dem Imprägnieren oder Tränken das Wasser durch Trocknen des imprägnierten bzw. getränkten Trägers entfernt wird. Es kann auch ohne vorherige Trocknung calciniert werden, wobei zu Beginn der Calcinierung das Lösungsmittel entweicht. Die Calcinierung des dotierten Trägers kann unter vermindertem Druck, unter Normaldruck oder unter erhöhtem Druck durchgeführt werden. Sie kann dabei sowohl in sauerstoffhaltiger Atmosphäre, als auch in Inertgasatmosphäre, wie unter Helium, Stickstoff oder Argon, oder unter Reaktivgasatmosphäre, wie unter Wasserstoff, Ammoniak, Kohlendioxid oder Kohlenmonoxid stattfinden.

Die Alkalimetalle werden in an sich bekannter Weise auf die, vorzugsweise dotierten, Träger aufgebracht. Hierzu gehört z.B. die Trägerung im schmelzflüssigen Zustand bei einer Temperatur im Bereich von 100 bis 300°C, wie sie in GB-A-1 143 993 beschrieben ist. Dazu wird die entsprechende Menge des Alkalimetalls als Strang oder Block zum Träger gegeben und unter Erwärmen mit ihm vermischt. Dabei erfolgt die feine Verteilung des Alkalimetalls auf dem Träger.

Ferner können die Alkalimetalle durch Imprägnierung mit Lösungen der Alkalimetallazide und anschließende thermische Zersetzung der Azide aufgebracht werden. Ein entsprechendes Verfahren ist beispielsweise in FR-A-2 609 024 beschrieben. Weiterhin können die Alkalimetalle durch Aufdampfen auf den Träger aufgebracht werden. Dies geschieht in der Regel unter vermindertem Druck.

Weiterhin können die Träger mit ammoniakalischen Lösungen der Alkalimetalle imprägniert werden, und der Ammoniak kann anschließend verflüchtigt werden.

Das Aufbringen der Alkalimetalle findet im Vakuum, unter Inertgasatmosphäre (He, $N_2$, As usw.) oder unter Reaktivgasatmosphäre ($H_2$, $CO_2$, CO) statt.

Die Katalysatoren werden in stark basisch katalysierten Reaktionen, vorzugsweise zur Seitenkettenalkylierung oder -alkenylierung von alkylaromatischen Verbindungen mit Olefinen oder Diolefinen, zur Doppelbindungsisomerisierung von Olefinen, zur Dimerisierung von Olefinen, zur Kern-Kern-Kopplung von Aromaten sowie zur Aminierung von Olefinen und konjugierten Diolefinen eingesetzt.

Dabei wird die Umsetzung im allgemeinen bei einer Temperatur von -50 bis 400°C, vorzugsweise von -20 bis 300°C, besonders bevorzugt von 80 bis 250°C, insbesondere von 100 bis 220°C und einem Druck vorzugsweise von 0,1 bis 200, besonders bevorzugt von 1 bis 150, insbesondere von 1 bis 100 bar durchgeführt.

Als alkylaromatische Verbindungen können dabei alle geeigneten Alkylaromaten eingesetzt werden. Sie können als aromatischen Kern beispielsweise einen Benzol- oder Naphthalinkern aufweisen. Weiterhin sind alkylalicyclische Verbindungen geeignet, in denen der cyclische Kern ein cyclischer Alkyl-, Alkenyl- oder Alkinylrest sein kann. Auch Reste, in denen mehrere der Ringstrukturen miteinander verknüpft sind, können eingesetzt werden. Die Ringstrukturen weisen in $\alpha$-Position der Seitenkette ein azides Wasserstoffatom auf. Vorzugsweise weisen sie mindestens einen Alkylrest auf, der an die cyclische Struktur gebunden ist. Die Alkylreste können dabei eine beliebige Länge aufweisen und durch weitere Substituenten substituiert sein. Vorzugsweise werden als alkylaromatische Verbindungen Benzole, die durch 1 bis 6, vorzugsweise 1 bis 3, insbesondere 1 bis 2 $C_{1-20}$-Alkylreste, vorzugsweise $C_{1-3}$-Alkylreste substituiert sind, Naphthaline, die durch 1 bis 10, vorzugsweise 1 bis 5, besonders bevorzugt 1 bis 2 $C_{1-20}$-, vorzugsweise $C_{1-3}$-Alkylreste substituiert sind, eingesetzt.

Die Olefine weisen vorzugsweise 2 bis 20, besonders bevorzugt 2 bis 10, insbesondere 2 bis 5 C-Atome auf Vorzugsweise werden Ethen, Propen, 1-Buten, 2-Buten, Isobuten, 1-Penten, 2-Penten, 2-Methyl-1-buten, 2-Methyl-2-buten und/oder 3-Methyl-1-buten eingesetzt. Besonders bevorzugt sind Ethen und Propen. Die Diolefine weisen vorzugsweise 4 bis 20, besonders bevorzugt 4 bis 10, insbesondere 4 bis 6 C-Atome auf. Besonders bevorzugt werden Butadien und/oder Isopren eingesetzt.

Besonders bevorzugt sind die Umsetzung von Toluol mit Ethen oder Propen zu Propylbenzol oder Isobutylbenzol, die Umsetzung von Cumol mit Ethen zu tert.-Amylbenzol und die Umsetzung von Xylolen mit Butadien zu 5-Tolylpentenen.

Bevorzugte Kern-Kern-Kopplungsreaktionen von Aromaten gehen von Benzol, Toluol, Ethylbenzol, Pyridin und Naphthalin aus. Bevorzugte Aminierungen sind die Aminierung von Ethen und Isopren.

Die Umsetzung kann diskontinuierlich oder vorzugsweise kontinuierlich in der Flüssig- oder Gasphase, bevorzugt in der Flüssigphase durchgeführt werden. Dabei können die bekannten Vorrichtungen zur Durchführung des Verfahrens eingesetzt werden.

Nachstehend wird die Erfindung anhand von Beispielen weiter erläutert.

**Beispiele**

**Herstellungsbeispiele**

Katalysator A (Vergleich)

$\gamma$-Al$_2$O$_3$ wurde mit 10 Gew.-% K$_2$CO$_3$ (gelöst in H$_2$O) imprägniert. Die Suspension wurde zur Trockene eingedampft und das so erhaltene Pulver bei 500°C im Luftstrom kalziniert. Das Material wurde dann im Vakuum bei 300°C während 3 h trocken gerührt. Zu diesem Pulver wurden 10 Gew.-% metallisches Natrium zugefügt und bei 300°C dispergiert.

Katalysator B (Vergleich)

Gefälltes ZrO$_2$•aq (entsprechend 37g ZrO$_2$) wurde mit 3,7 g KOH naß imprägniert und 16 h bei 300°C getrocknet. Auf 10 g des so erhaltenen Pulvers wurde 1 g Na zugefügt und bei 300°C dispergiert.

Katalysator C (Vergleich)

30 g TiO$_2$ (Rutil, Hersteller BASF AG) wurden mit 3 g K$_2$CO$_3$ naß imprägniert und 16 h bei 500 °C calciniert. Auf 10 g des so erhaltenen Pulvers wurde 1 g Na zugefügt und bei 300°C dispergiert.

Katalysator D

Gefälltes ZrO$_2$•aq (entsprechend 6,16 g ZrO$_2$) wurde mit 33,3 ml einer 1,5-molaren Ba(OAc)$_2$-Lösung imprägniert, die Suspension zur Trockene eingedampft, bei 150°C getrocknet und 33 h bei 800°C calciniert. Auf 10 g des so erhaltenen BaZrO$_3$ (röntgendiffraktometrisch bestimmt) wurde anschließend aus wäßriger Lösung 1 g K$_2$CO$_3$ aufgebracht, bei 350°C getrocknet und zu diesem Pulver schließlich 1 g metallisches Natrium zugefügt und bei 300°C dispergiert.

Katalysator E

In 100 ml einer zweimolaren Mg(OAc)$_2$-Lösung wurden 45,6 g Ti(OEt)$_4$ eingerührt, die erhaltene Suspension zur Trockene eingedampft, bei 150°C getrocknet und 33 h bei 800°C calciniert. Auf 10 g des so erhaltenen MgTiO$_3$ (röntgendiffraktometrisch bestimmt) wurden anschließend aus wäßriger Lösung 1 g K$_2$CO$_3$ aufgebracht, bei 400°C getrocknet und zu diesem Pulver anschließend 1 g metallisches Natrium zugefügt und bei 300°C dispergiert.

Katalysator F

Gefälltes ZrO$_2$•aq (entsprechend 12,32 g ZrO$_2$) wurde mit 100 ml einer 1-molaren Ca(OAc)$_2$-Lösung imprägniert, die Suspension zur Trockene eingedampft, bei 150°C getrocknet und 33 h bei 800°C calciniert. Auf 10 g des so erhaltenen CaZrO$_3$ (röntgendiffraktometrisch bestimmt) wurden anschließend aus wäßriger Lösung 1 g K$_2$CO$_3$ aufgebracht, bei 400°C getrocknet und zu diesem Pulver anschließend 1 g metallisches Natrium zugefügt und bei 300°C dispergiert. Katalysator G

12,1 g Ca wurden in 400 ml EtOH gelöst und anschließend 68,37 g Ti(OEt)$_4$ zugegeben. Die erhaltene Ethanolatlösung wurde mit einer Mischung von 30 g H$_2$O und 50 g EtOH unter starkem Rühren geliert, 1 h gerührt, bei 50°C um Vakuum getrocknet und 5 h bei 600°C calciniert. Auf 10 g des so erhaltenen Perowskits CaTiO$_3$ (röntgendiffraktometrisch bestimmt) wurden anschließend aus wäßriger Lösung 1 g K$_2$CO$_3$ aufgebracht, bei 400°C getrocknet und zu diesem Pulver anschließend 1 g metallisches Natrium zugefügt und bei 300°C dispergiert.

**Verfahrensbeispiele**

Vergleichsbeispiele V1 - V3

10 g Katalysator A bis C wurden mit 85 g Toluol in einem druckfesten Reaktionsgefäß vorgelegt. Nach Zugabe von 20 g Propen wurde das Reaktionsgefäß auf 160°C aufgeheizt und die Reaktionssuspension anschließend 12 h lang gerührt. Die Ergebnisse sind in Tabelle 1 aufgeführt.

Beispiele 1 bis 4

10 g der Katalysatoren D bis G wurden mit 85 g Toluol in einem druckfesten Reaktionsgefäß vorgelegt. Nach Zugabe von 20 g Propen wurde das Reaktionsgefäß auf 160°C aufgeheizt und die Reaktionssuspension anschließend 12 h lang gerührt. Die Ergebnisse sind in Tabelle 1 aufgeführt.

Tabelle 1

|  | Katalysator | bezüglich Propen | | | bezüglich Toluol | |
|---|---|---|---|---|---|---|
|  |  | U | $S_{iBB}$ | $S_{MePenten}$ | U | $S_{iBB}$ |
| Vergleichsbeispiel V1 | A | 39 | 55 | 40 | 12 | 92 |
| Vergleichsbeispiel V2 | B | 36 | 59 | 36 | 11 | 93 |
| Vergleichsbeispiel V3 | C | 3 | 63 | 27 | 1 | 86 |
| Beispiel 1 | D | 60 | 70 | 20 | 24 | 87 |
| Beispiel 2 | E | 46 | 74 | 19 | 19 | 90 |
| Beispiel 3 | F | 39 | 70 | 21 | 16 | 88 |
| Beispiel 4 | G | 52 | 72 | 19 | 21 | 89 |

U = Umsatz [mol%]
$S_{iBB}$ = Selektivität für Isobutylbenzol [mol%]
$S_{MePenten}$ = Selektivität für Methylpenten [mol%]

**Patentansprüche**

1.  Katalysator, enthaltend mindestens ein Alkalimetall auf einem Träger charakterisiert durch die allgemeine Formel (I)

$$A_{n'}Ti_{m'}Zr_{p'}Hf_{q'}O_{n'+2(m'+p'+q')} \tag{I}$$

mit der Bedeutung von
A zweiwertiges Metall,

$$20 \cdot (m'+p'+q') > n' > 0{,}05 \cdot (m'+p'+q'),$$

der mit mindestens einer Verbindung eines Alkalimetalls und/oder Erdalkalimetalls dotiert sein kann, wobei das Gewichtsverhältnis Alkalimetall/Träger 0,01 bis 5 zu 1 und bei Vorliegen einer Dotierung das Gewichtsverhältnis Dotierung/Träger 0,01 bis 5 zu 1 beträgt und wobei der Phasenanteil des Trägers, der einer $ZrO_2$- oder einer Erdalkalioxid-Struktur entspricht oder aus $ZrO_2$ oder Erdalkalioxid besteht, kleiner als 10 Gew.-% beträgt.

2.  Katalysator nach Anspruch 1, dadurch gekennzeichnet, daß der Träger - im undotierten Zustand - aus komplexen Oxiden von zweiwertigen Metallen A und vierwertigem Titan (Ti), Zirkonium (Zr) und/oder Hafnium (Hf) besteht.

3.  Katalysator nach Anspruch 2, dadurch gekennzeichnet, daß die Oxide der allgemeinen Formeln $ATi_mO_{1+2m}$, $A_nTi_mO_{n+2m}$, $AZr_pO_{1+2p}$, $A_nZr_pO_{n+2p}$, $ATi_mZr_pO_{1+2(m+p)}$, $A_nTi_mZr_pO_{n+2(m+p)}$ enthalten sind, mit der Bedeutung von n, m, p, q als ganze Zahlen.

4.  Katalysator nach einem der Ansprüche 1 bis 3, dadurch gekennzeichnet, daß A ausgewählt ist aus Mg, Ca, Sr, Ba, Mn, Fe, Co, Ni, Zn, Cd, Pb und Gemischen davon.

5.  Katalysator nach einem der Ansprüche 1 bis 4, dadurch gekennzeichnet, daß er zwingend mit mindestens einem Hydroxid oder Carbonat eines Alkalimetalls und/oder Erdalkalimetalls dotiert ist.

6.  Verfahren zur Herstellung eines Katalysators gemäß einem der Ansprüche 1 bis 5 durch

- Aufbringen mindestens eines Alkalimetalls auf den Träger der allgemeinen Formel (I) durch
  Trägerung des schmelzflüssigen Alkalimetalls, oder
  Imprägnierung oder Tränkung des Trägers mit Lösungen eines Alkalimetallazids, Trocknen des Trägers und
  Zersetzung des Alkalimetallazids, oder
  Aufdampfen des Alkalimetalls auf den Träger, oder
  Imprägnierung oder Tränkung des Trägers mit ammoniakalischen Lösungen des Alkalimetalls und Entfernen
  des Ammioniaks,

- wobei der Träger der allgemeinen Formel (I) gegebenenfalls vorher dotiert wurde durch Imprägnieren oder
  Tränken mit einer Lösung mindestens einer Verbindung eines Alkalimetalls und/oder Erdalkalimetalls, Trocknen und Kalzinieren des dotierten Trägers.

7. Verwendung eines Katalysators, wie er in einem der Ansprüche 1 bis 5 definiert ist, in stark basisch katalysierten Reaktionen, vorzugsweise zur Seitenkettenalkylierung oder -alkenylierung von alkylaromatischen Verbindungen mit Olefin oder Diolefinen, zur Doppelverbindungsisomerisierung von Olefinen, zur Dimerisierung von Olefinen, zur Kern-Kern-Kopplung von Aromaten sowie zur Aminierung von Olefinen und konjugierten Diolefinen.

8. Verfahren zur Seitenkettenalkylierung oder -alkenylierung von alkylaromatischen Verbindungen durch Umsetzung mit Olefinen oder Diolefinen, dadurch gekennzeichnet, daß die Umsetzung in Gegenwart eines Katalysators durchgeführt wird, wie er in einem der Ansprüche 1 bis 5 definiert ist.

9. Verfahren nach Anspruch 8, dadurch gekennzeichnet, daß die Umsetzung bei einer Temperatur im Bereich von - 50 bis 400°C und einem Druck im Bereich von 0,1 bis 200 bar durchgeführt wird.

10. Verfahren nach einem der Ansprüche 8 oder 9, dadurch gekennzeichnet, daß als alkylaromatische Verbindungen Benzole, die durch 1 bis 6 $C_{1-20}$-Alkylreste substituiert sind und Naphthaline, die durch 1 bis 10 $C_{1-20}$-Alkylreste substituiert sind, eingesetzt werden.

11. Verfahren nach einem der Ansprüche 8 bis 10, dadurch gekennzeichnet, daß als Olefine Ethen, Propen, 1-Buten, 2-Buten, Isobuten, 1-Penten, 2-Penten, 2-Methyl-1-buten, 2-Methyl-2-buten und/oder 3-Methyl-1-buten, und als Diolefine Butadien oder Isopren eingesetzt werden.

12. Verfahren nach einem der Ansprüche 8 bis 11, dadurch gekennzeichnet, daß es in der Flüssig- oder Gasphase kontinuierlich durchgeführt wird.